# EUROPEAN PATENT APPLICATION

(11) **EP 1 293 131 A1**
(43) Date of publication of application: **19.03.2003**
(21) Application number: 01941120.6
(22) Date of filing: 20.06.2001
(51) Int. Cl.: A23G 3/00, A61K 31/19, A61K 31/352, A61K 31/405, A61K 31/722, A61K 33/10, A61K 35/12, A61K 35/64, A61K 35/78, A61P 11/04, A61P 31/04, A23L 1/30, A23L 2/00

(54) **THROAT CARE AGENTS**

(30) Priority: 21.06.2000 JP 2000186112; 07.12.2000 JP 2000372791
(71) Applicant: Kao Corporation, Tokyo 103-8210 (JP)
(72) Inventor: KAYANE, Shigeto, c/o KAO CORPORATION RES. LAB., Tokyo 131-8501 (JP); ISOBE, Tsutomu, c/o KAO CORPORATION RES. LAB., Tokyo 131-8501 (JP); IWASE, Tadayuki, c/o KAO CORPORATION RES. LAB., Tokyo 131-8501 (JP); MIURA, Yasuhiro, c/o KAO CORPORATION RES. LAB., Tokyo 131-8501 (JP)
(74) Representative: VOSSIUS & PARTNER
(86) International application number: JP0105260
(87) International publication number: WO01097627

(57) **Abstract**

Provided is a throat care agent which promotes the secretion of the saliva, promoting the secretion of a large amount of mucin which is a bactericidal component contained in the saliva, inhibiting the adhesion of pathogenic bacteria to the throat, and removing them from the throat to keep it refreshed. A throat care agent comprising a component promoting the secretion of the saliva.

## Description

### TECHNICAL FIELD

The present invention relates to a throat care agent capable of promoting the secretion of the saliva, sterilizing and cleaning the throat, preventing a bacterial infection of the pharyngeal mucosa and keeping the throat refreshed.

### BACKGROUND ART

As an oral care agent, proposed are a xylitol-containing edible composition for promoting the secretion of the saliva of patients suffering from xerostomia, thereby relaxing the symptoms in the oral cavity (Japanese Patent Laid-Open No. Hei 3-83920) and a halitosis inhibiting composition containing both an oral deodorant derived from plants of the *Labiatae* family and a saliva secretion promoter such as plants of the *Sterculiaceae* Family.

Their aim however exists in the treatment of dry mouth, enhancement of the halitosis preventing effect or prolongation of duration, not in protection from the bacteria attached to the throat which will otherwise produce various symptoms of, for example, cold or alleviation of such symptoms.

Various lozenges containing an antitussive and expectorant, a bactericide, an anti-inflammatory or the like and acting on the throat, thereby preventing inflammatories of the throat or cough are popularly employed. These lozenges however have a limitation in their effect, because their medicinal effect is brought about by a component not existing in the human body.

An object of the present invention is to provide a throat care agent capable of protecting the throat from infection with a cold virus or secondary bacterial infection, in other words, sterilizing and cleaning the throat, and activating the body's self protective reaction, thereby efficiently caring the throat or alleviating symptoms due to such infection.

### DISCLOSURE OF THE INVENTION

Paying attention to the body's self protective function of the saliva, the present inventors have carried out an investigation and as a result, have found that a large amount of mucin contained in the saliva is effective for trapping (including in the mucin) inflammation-causing bacteria adversely affecting the throat or eliminating such inflammation-causing bacteria adhered to the throat.

The present inventors also paid attention to that the proliferation cycle of a cold virus occurs not in the oral mucosa but only in the respiratory mucosal epithelium ("Saibo Kogaku", 19(1), 33-38) and proliferation and activation of a cold virus is enhanced by a protease produced by bacteria such as *Haemophilus influenzae, Pseudomonas aeruginosa* or *Staphylococcus aureus* ("Modern Physician", 18(11), 1339-1342). As a result of studying inhibition of such bacteria from adhering to the throat and removal of them, they have found that the saliva exhibits an action of preventing adhesion of such bacteria to the throat or a bacteria removing action, and eliminating a throat discomfort.

In one aspect of the present invention, there is thus provided a throat care agent containing a saliva-secretion promoting component. The throat care agent containing, in addition, a polysaccharide; and the throat care agent further containing a throat acting component are also provided.

In another aspect of the present invention, there is also provided a throat caring method, which comprises administering a saliva-secretion promoting component.

In a further aspect of the present invention, there is also provided use of a saliva-secretion promoting component for the preparation of a throat care agent.

In a still further aspect of the present invention, there is also provided a throat care agent in the form of a lozenge or beverage.

### BRIEF DESCRIPTION OF THE DRAWINGS

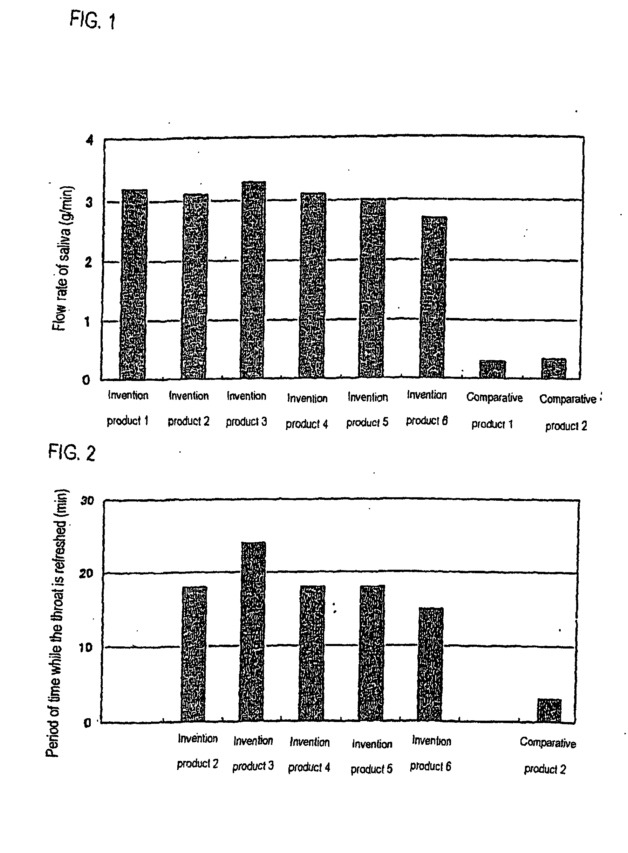

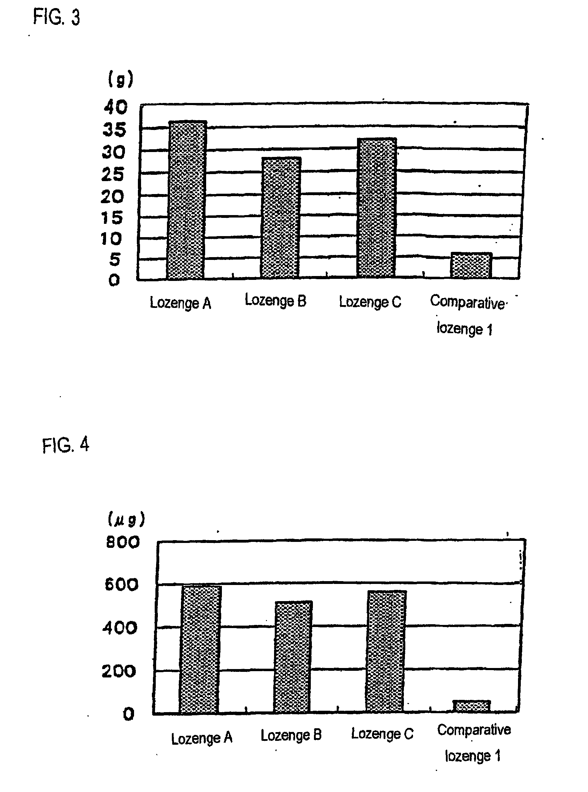

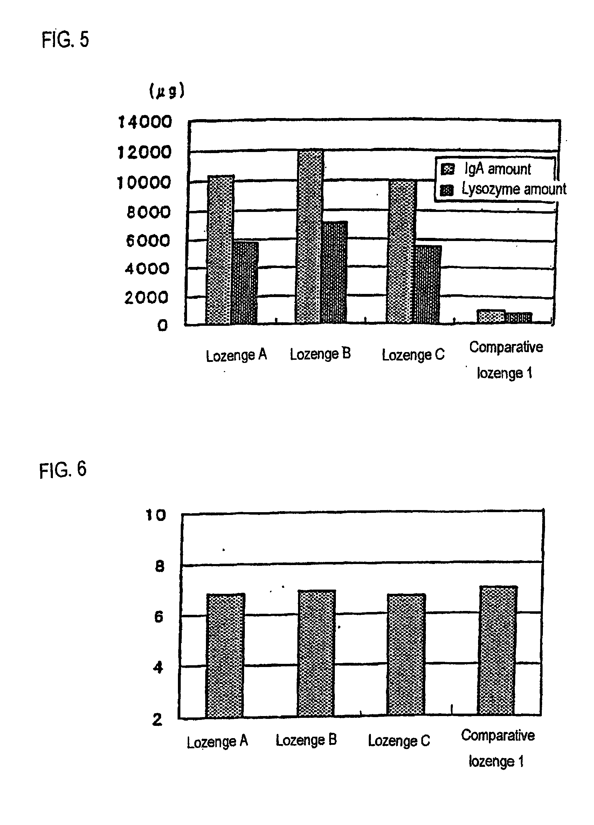

FIG. 1 illustrates the saliva flow rate when a throat care agent is administered; FIG. 2 illustrates the duration of refreshing feeling when a throat care agent is administered; FIG. 3 illustrates the secretion amount of the saliva stimulated by a lozenge; FIG. 4 illustrates a mucin content of the saliva when stimulated by a lozenge; FIG. 5 illustrates a content of a bactericidal component in the saliva when stimulated by a lozenge; and FIG. 6 illustrates pH of the oral saliva when stimulated by the saliva.

### THE BEST MODE FOR CARRYING OUT THE INVENTION

Examples of the saliva-secretion promoting component usable in the present invention include a taste stimulating component, an oral mucosa stimulating component, a salivary gland activating component and a physically stimulating component. The term "saliva-secretion promoting component" as used herein means a component having 1.2 or greater, preferably 2 or greater, more preferably 5 or greater, as a ratio - relative to a saliva secretion rate of a healthy person measured in a resting state within 30 minutes after wake-up without eating anything - of a saliva secretion rate after taking the saliva-secretion promoting component in a similar resting state, just after taking the taste stimulating component or oral mucosa stimulating substance, 60 to 180 minutes after taking the salivary gland activating component, or just after taking the physically stimulating component.

Examples of the taste stimulating component include organic acids and flavoring substances.

Examples of the organic acids include citric acid, isocitric acid, malic acid, acetic acid, oxalic acid, malonic acid, succinic acid, glutaric acid, maleic acid, fumaric acid, aconitic acid, lactic acid, tartaric acid, pyruvic acid, ascorbic acid, aldonic acid, and uronic acid. In addition, plum vinegar, apple vinegar, and chips, powder or extract of citrus such as lemon, orange, citron, or Chinese citron can be given as examples.

Examples of the flavoring substance include amino acids (salts), nucleic acids, dipeptides, tripeptides, and oligopeptides, more specifically, sodium glutamate, inosinic acid and guanylic acid. Of these, sodium glutamate and inosinic acid are preferred.

Examples of the oral mucosa stimulating component include isothiocyanates, amide-series pungent components, vanillyl ketones, more specifically, allyl isothiocyanate, capsaicin, piperine, sanshool, and zingerol.

Examples of the salivary gland activating component include parasympathomimetic agents, cholinesterase inhibitors, and calcium ion releasing agents, more specifically, pilocarpine, muscarine, acetylcholine, ryanodine, caffeine, and plants such as *Cola acuminata, Cola nitida,* jaborandi, white birch, honeysuckle, American ginseng, *houttuynia cordata,* garlic, hibiscus, hop, *Actinidia polygama,* linden and rose hip.

Of the above-described components, those exhibiting volatility by themselves such as allyl isothiocyanate and sanshool have also an action of stimulating a sense of smell and promoting the secretion of the saliva.

Examples of the physically stimulating component include a effervescent and a carbon dioxide gas, nitrogen gas or inactive gas filled under high pressure.

As the effervescent, a combination of an organic acid and a carbonate or bicarbonate is preferred. Examples of the organic acid include citric acid, tartaric acid, ascorbic acid, malic acid, fumaric acid, succinic acid and malonic acid, while those of the carbonate or bicarbonate include sodium carbonate, sodium bicarbonate, potassium carbonate, potassium bicarbonate, ammonium carbonate and sodium sesquicarbonate. Of these, sodium carbonate, sodium bicarbonate and potassium carbonate are especially preferred. The term "gas filled under high pressure" means a gas dissolved in an aqueous solution or that filled in the voids of a solid.

Examples of the inactive gas include argon, neon and krypton.

As the saliva-secretion promoting component, preferred are tartaric acid, allyl isothiocyanate, caffeine, an effervescent composed of tartaric acid and sodium bicarbonate and an effervescent composed of citric acid and sodium bicarbonate.

These saliva-secretion promoting components may be used either singly or in combination.

The throat care agent of the present invention has improved effects when the saliva-secretion promoting component is used in combination with a polysaccharide.

Examples of the polysaccharide include soybean dietary fiber, guar gum, tara gum, tragacanth gum, gum arabic, xanthan gum, karaya gum, gellan gum, corn dietary fiber, alginic acid, cherry gum, mesquite gum, locust bean gun, agar and phosphoric acid polysaccharide. Of these, soybean dietary fiber, guar gum, tara gum, locust bean gum and phosphoric acid polysaccharide are preferred. As the phosphoric acid polysaccharide, phosphoric acid mannan is especially preferred.

These polysaccharides may be used in combination. For example, combinations of phosphoric acid mannan - soybean dietary fiber, soybean dietary fiber - tara gum, and phosphoric acid mannan - tara gum are preferred.

A content weight ratio of the saliva-secretion promoting component and polysaccharide, that is, saliva-secretion promoting component/polysaccharide preferably ranges from 100/1 to 1/100, especially 10/1 to 1/10.

The throat care agent of the present invention has improved effects for relaxing a throat discomfort and heightening refreshed feeling of the throat when using the saliva-secretion promoting component in combination with a throat acting component.

Examples of the throat acting component include a herb, an antitussive and expectorant, chitin, chitosan, honey, propolis and a salivary component.

The herb is an edible or medicinal plant having, at its leaves, petals or stems, a flavor. Specific examples include spearmint, peppermint, wintergreen, sassafras, clove, sage, eucalyptus, *Origanum marjorana,* cinnamon, thyme, tea tree, perilla, echinacea, *Rubus suauissmus,* loquat honey, *Pseudocydonis cinensis*, Korean ginseng, ganoderma, kumquat, Japanese radish, tansy, saponin, loquat, great plantain, fennel, ginger, ginkgo, saffron and jujube, and chips, powder, juice and extracts thereof. Components in these herbs such as menthol, cineol, anethole and carvone may be used instead. In this case, menthol and cineol are preferred.

As the antitussive and expectorant, dextromethorphan phenolphthalin salt, methylephedrine hydrochloride, potassium guaiacolsulfonate, guaiphenesin, potassium cresolsulfonate, cetylpyridinium chloride, dequalinium chloride, chlorhexidine hydrochloride, and noscapine are especially preferred.

Examples of the salivary component, mucin, secretory IgA, secretory IgM, lysozyme, lactoferrin, peroxidase, kallikrein, fucose-rich glycoprotein, fibronectin, proline-rich glycoprotein, α-amylase, statherin, histidine-rich protein, invertase, dextranase, glycosidase, estrogen, progestin, androgen, corticosteroid, thyroxine and melatonin.

These throat acting components may be used in combination.

As the throat acting component, herbs, herb extracts, chitosan, honey, propolis and cineol are preferred, because they impart the throat with cooling and refreshing feelings.

When the saliva-secretion promoting component and throat acting component are used in combination, it is preferred to add the former one in an amount of 0.05 to 25 wt.%, preferably 1 to 15 wt.% and the latter one in an amount of 0.01 to 5 wt.%, preferably 0.5 to 2 wt.%, each in the throat care agent of the present invention.

The longer the throat care agent of the present invention stays on the throat, the more effectively durability of the throat acting component and secretion of the saliva are promoted. Viscous beverages such as syrup, jelly beverage and soft drink are therefore excellent in their effects.

From the viewpoint of better swallowability, the viscosity of such a beverage, as measured at 20°C by a Brookfield viscometer, preferably ranges from 2 to 3000 mPa·s, more preferably from 50 to 1800 mPa·s, especially from 150 to 400 mPa·s. Examples of a thickener for regulating the viscosity include starch syrup, sucrose, pectin, carrageenan, locust bean gum, guar gum and gelatin. When the thickener is employed, use of water as a base is especially preferred.

In addition, the throat care agent of the present invention is preferably in the form of a lozenge such as drop or candy. Lozenges composed mainly of sugar and/or starch syrup are preferred, of which those obtained by boiling down at high temperature (140 to 180°C) are particularly preferred. Lozenges may be sugar-free type composed mainly of a sugar alcohol.

The effervescent component may be incorporated substantially uniformly in the throat care agent of the present invention in the lozenge form. Alternatively, the lozenge may be formed to have an effervescent-containing portion and an effervescent-free portion so that the dissolution of the former finishes prior to that of the latter.

The effervescent-containing portion may be distinguished from the effervescent-free portion by forming the lozenge to have a laminate structure, for example, of an upper layer and a lower layer, or an upper layer, an intermediate layer and a lower layer. The lozenge may be a coated type having an inner layer and an outer layer with which the periphery of the inner layer is covered. The lozenge may be partially formed of blocks, columns or spheres.

A ratio (weight ratio) of the effervescent-containing portion to the effervescent-free portion preferably ranges from 0.1:1 to 1:0.1, especially from 0.4:1 to 1:0.4. When the lozenge has these two portions, the content of the effervescent component in the effervescent-containing portion is preferably from 1 to 80 wt.%, more preferably from 5 to 60 wt.%, especially from 10 to 40 wt.%, in terms of the total of an organic acid and a carbonate or bicarbonate, in consideration of the foaming property. When the lozenge has these two portions, the throat acting component may be incorporated in both of them or either one of them.

The throat lozenge is preferably prepared in a conventional manner when it has a substantially uniform structure. When it has a laminate structure, it is preferred to prepare the effervescent-containing portion and the effervescent-free portion separately and then stack them one after another. The coated type lozenge is preferably obtained by preparing the effervescent-free portion first and then covering it with the effervescent-containing portion.

The throat lozenge of the present invention is preferred, because it has thus excellent effervescence and saliva-secretion promoting effect.

When the throat care agent contains citric acid as the saliva-secretion promoting component and sodium bicarbonate as the effervescent, it is administered preferably in an amount of 10 g or greater/adult (60 g in weight)/day. When it is in the form of a throat lozenge, 5 g/piece of it is preferably administered at least two times a day in order to prevent adhesion of bacteria to the throat, clean the throat by removing them if any, and keep the throat refreshed.

When the saliva is excellent in sterilizing and cleaning effects and the throat acting component is added to such saliva, more improved throat care effect is available by the synergistic action of the salivary component and another component.

### Examples

Bacterial removal by the saliva was analyzed by the below-described test.

### Test 1: Bacteria trapping effect of the saliva

Bacterial solutions containing 10⁸ /mL, in viable cell count, of *Staphylococcus aureus* and *Haemophilus influenzae* were prepared using PBS, respectively. Each of the bacterial solutions and an equal amount of the saliva to be tested were mixed for 30 minutes. The human pharyngeal epithelial cells were washed with PBS to prepare a cell solution containing 2.5 x 10⁴ /mL of cells. Then, 0.02 mL of the bacterial solution mixed with the test solution was mixed with 0.98 mL of the cell solution, followed by reaction at 37°C for 30 minutes. The reaction mixture was subjected to centrifugal separation at 600 r/min for 10 minutes. The centrifugate was washed with PBS three times. The washed centrifugate was sprayed onto a plate, followed by gram staining. By an optical microscope at a predetermined magnification, the number of bacteria within the field of the microscope was counted. The percentage of the bacteria adhered to the cells was counted supposing that the number of bacteria under control conditions was 100%. Under the control conditions, PBS was used as the test solution. As a result, owing to the trapping effects of the saliva, the percentages of *Staphylococcus* aureus and *Haemophilus influenzae* were 10% and 5%, respectively.

### Test 2: Bacteria coating effects of the saliva

Bacterial solutions containing 10⁸ /mL, in viable cell count, of *Staphylococcus aureus* and *Haemophilus influenzae* were prepared, respectively, with PBS. The human pharyngeal epithelial cells were washed with PBS to prepare a solution containing 2.5 x 10⁴ /mL of the cells. The resulting solution was mixed with an equal amount of the saliva to be tested and they were reacted. The reaction mixture was then subjected to centrifugal separation at 600 r/min for 10 minutes. The centrifugate was washed three times with PBS to prepare a cell solution. With 0.02 mL of each bacterial solution, 0.98 mL of the cell solution was mixed and they were reacted at 37°C for 30 minutes. The reaction mixture was subjected to centrifugal separation at 600 r/min for 10 minutes. The centrifugate was washed three times with PBS. The washed centrifugate was sprayed onto a plate, followed by gram staining. By an optical microscope at a predetermined magnification, the number of bacteria within the field of the microscope was counted. The percentage of the bacteria adhered to the cells was counted supposing that the number of bacteria under control conditions was 100%. Under the control conditions, PBS was used as the test solution. As a result, owing to the coating effect of the saliva, the percentage of *Staphylococcus aureus* and *Haemophilus influenzae* were 20% and 10%, respectively.

### Test 3: Bacteria trapping effect and coating effect of mucin.

Tests 1 and 2 were conducted using, as the test solution, a solution (0.001%) of human-derived mucin. Owing to its trapping effects, the percentages of the *Staphylococcus aureus* and *Haemophilus influenzae* were 12% and 5%, respectively, while owing to its coating effects, the percentages of *Staphylococcus aureus* and *Haemophilus influenzae* were 22% and 10%, respectively.

### Example 1

Throat care agents each having the following composition were prepared.

**Table 1**

| | | | | | | | | |
|---|---|---|---|---|---|---|---|---|
| g | | | | | | | | |

| | Invention products | | | | | | Comparative Products | |
|---|---|---|---|---|---|---|---|---|
| | 1 | 2 | 3 | 4 | 5 | 6 | 1 | 2 |
| Saliva-secretion promoting component | | | | | | | | |
| Tartaric acid | 1 | 1 | 1 | - | - | 1 | - | - |
| Allyl isothiocyanate | - | - | - | 1 | - | - | - | - |
| Caffeine | - | - | - | - | 1 | - | - | - |

| Throat acting component | | | | | | | | |
|---|---|---|---|---|---|---|---|---|
| Menthol | - | 1 | 1 | 1 | 1 | 1 | - | 1 |

| Thickening component | | | | | | | | |
|---|---|---|---|---|---|---|---|---|
| Starch syrup Gelatin Sucrose The other component water | 86 | 85 | 98 | 85 | 85 | - | 86 | - |
| | - | - | - | - | - | 10 | - | - |
| | - | - | - | - | - | 48 | - | - |
| | 13 | 13 | - | 13 | 13 | 40 | 14 | 99 |
| Viscosity 20°C mPa·s | 480 | 480 | 2800 | 450 | 430 | 320 | 470 | 1.3 |

The secretion flow rate of the saliva when each of the throat care agents shown in Table 1 was administered was measured in the following manner.

### Measurement of the secretion flow rate of the saliva

Ten healthy adults were administered with 10 g of the throat care agent shown in Table 1. Just after administration of each of Invention products 1, 2, 3, 4 and 6, and Comparative products 1 and 2, or 90 minutes after administration of Invention product 5, the saliva was wiped off well from the mouth by absorbent cotton. They were asked to discharge, from the mouth, the whole amount of the saliva secreted for 5 minutes after the wiping-off. This weight was regarded as the secretion flow rate of the saliva. Each product was evaluated based on an arithmetic mean of ten subjects.

The results are shown in FIG. 1. It has been understood that the secretion flow rate of the saliva when each of Invention products 1 to 6 was administered was 10 times as much as that when Comparative products were administered, suggesting promotion of the secretion of the saliva.

### Example 2

Ten adults who caught a cold and therefore had some throat discomfort were administered with 10 g of each of Invention products 2 to 6 and Comparative product 2 shown in Table 1 and a time from just after administration until disappearance of the refreshed feeling was measured. Evaluation was conducted based on an arithmetic mean of 10 adults.

The results are shown in FIG. 2. It has been understood that refreshed feeling of the throat lasted for longer minutes when Invention products 2 to 6 were administered. Especially, the refreshed feeling lasted for about 24 minutes when Invention product 3 having a high viscosity was administered.

### Example 3

Lozenges having the following composition were prepared.

| | |
|---|---|
| Citric acid | 12.5 wt.% |
| Sodium bicarbonate | 12.5 |
| Starch syrup (solid content) | 15 |
| Palatinit | 59.5 |
| Menthol | 0.5 |

Ten adults were administered with 5 g of this lozenge. They were asked to discharge from their mouth the whole amount of the saliva secreted until the disappearance of the lozenge. The value obtained by subtracting, from this amount, the weight of the lozenge was regarded as the secretion amount of the saliva. Evaluation was conducted based on the arithmetic mean of these ten adults.

The saliva secretion amount when the lozenge of the present invention was used was 52 g.

The bacteria trapping effect and the bacteria coating effect of the resulting lozenge were analyzed in the following test method.

### Test 1: Bacteria trapping effect

Bacterial solutions containing 10⁸ /mL, in viable cell count, of *Staphylococcus aureus* and *Haemophilus influenzae* were prepared, respectively, with PBS. Each of the bacterial solutions and an equal amount of the saliva to be tested were mixed for 30 minutes. The human pharyngeal epithelial cells were washed with PBS (phosphate buffer solution) to prepare a cell solution containing 2.5 x 10⁴ /mL of cells. Then, 0.02 mL of the bacterial solution mixed with the test solution was mixed with 0.98 mL of the cell solution, followed by reaction at 37°C for 30 minutes. The reaction mixture was subjected to centrifugal separation at 600 r/min for 10 minutes. The centrifugate was washed three times with PBS. The washed centrifugate was sprayed onto a plate, followed by gram staining. By an optical microscope at a predetermined magnification, the number of the bacteria within the field of the microscope was counted. The percentage of the bacteria adhered to the cells was measured supposing that the number of the bacteria under control conditions was 100%. Under the control conditions, PBS was used as the test solution.

### Test 2: Bacteria coating effect

Bacterial solutions containing 10⁸ /mL, in viable cell count, of *Staphylococcus aureus* and *Haemophilus influenzae* were prepared, respectively, with PBS. The human pharyngeal epithelial cells were washed with PBS to prepare a solution containing 2.5 x 10⁴ /mL of cells. The resulting solution was mixed with an equal amount of a test solution and they were reacted. The reaction mixture was subjected to centrifugal separation at 600 r/min for 10 minutes. The centrifugate was washed three times with PBS to prepare a cell solution. With 0.02 mL of each bacteria solution, 0.98 mL of the cell solution was mixed and they were reacted at 37°C for 30 minutes. The reaction mixture was subjected to centrifugal separation at 600 r/min for 10 minutes, followed by washing with PBS three times. The washed centrifugate was sprayed onto a plate, followed by gram staining. By an optical microscope at a predetermined magnification, the number of bacteria within the field of the microscope was counted. The percentage of bacteria adhered to the cells was found supposing that the number of the bacteria under control conditions was 100%. Under the control conditions, the PBS solution was used as the test solution.

Owing to the trapping effect and coating effect of a mixture of the saliva secreted, stimulated by the lozenge of the invention and the dissolved lozenge, percentages of *Staphylococcus aureus* adhered to the cells were 10% and 20%, respectively. Owing to the trapping effect and coating effect, the percentages of *Haemophilus influenzae* adhered to the cells were, on the other hand, 5% and 10%, respectively.

### Example 4

Lozenges having the following composition were prepared.

| | |
|---|---|
| Citric acid | 12.5 wt.% |
| Sodium bicarbonate | 12.5 |
| Soybean dietary fiber | 0.5 |
| Starch syrup (solid content) | 15 |
| Palatinit | 59 |
| Menthol | 0.5 |

As a result of measurement in a similar manner to Example 3, the secretion amount of the saliva, stimulated by the above-described lozenge of the present invention was 52 g. On the other hand, in the case of a lozenge (comparison) containing 20 wt.% of starch syrup, 79.5 wt.% of palatinit and 0.5 wt.% of menthol, the secretion amount of the saliva was 5 g.

The bacteria trapping effect and coating effect of a mixture of the saliva secreted, stimulated by the lozenge of the invention and the dissolved lozenge were analyzed as in Example 3. The percentages adhered to the cells were 0% and 0%, respectively. With regards to the bacteria trapping effect and coating effect when the lozenge (comparison) was used, the percentages were 100% and 100%, respectively.

### Example 5

A two-layer type lozenge having an outer layer and an inner layer at a weight ratio of 7:3 was prepared.

| Outer layer: | |
|---|---|
| Tartaric acid | 12.5 wt.% |
| Sodium bicarbonate | 12.5 |
| Starch syrup (solid content) | 16 |
| Palatinit | 59 |

| Inner layer: | |
|---|---|
| Starch syrup (solid content) | 20 wt.% |
| Palatinit | 78.5 |
| Soybean dietary fiber | 1 |
| Menthol | 0.5 |

Evaluation was conducted as in Example 3. With regards to the above-described lozenge, the secretion amount of the saliva was 43%, the percentage as a result of the test on bacteria trapping effect was 0% and the percentage as a result of the test on bacteria coating effect was 0%.

### Example 6

A syrup having the following composition was prepared.

| | |
|---|---|
| Starch syrup | 95.5 wt.% |
| Menthol | 1 |
| Soybean dietary fiber | 1 |
| Guar gum | 1 |
| Caffeine | 0.5 |
| Polyglycerin fatty acid ester | 1 |

Ninety minutes after ten adults took 5 g of the syrup thus obtained, they were asked to wipe off the saliva in the mouth well by absorbent cotton and then, to discharge, from the mouth, the whole amount of the saliva secreted for 5 minutes after the wiping-off. This weight was regarded the secretion amount of the saliva. The syrup was evaluated based on an arithmetic mean of ten subjects.

The secretion amount of the saliva was 17 g. With regards to the bacteria trapping effect and the bacteria coating effect evaluated as in Example 3, the percentages were each 0%.

On the other hand, when a beverage (comparison) containing 98 wt.% of a starch syrup, 1 wt.% of menthol and 1 wt.% of polyglycerin fatty acid ester was administered, the saliva secretion amount was 2 g; and with regards to the bacteria trapping effect and the bacteria coating effect, the percentages were each 100%.

### Example 7

A beverage having the following composition was prepared.

| | |
|---|---|
| Water | 95.5 wt.% |
| Menthol | 1 |
| Soybean dietary fiber | 1 |
| Guar gum | 1 |
| Caffeine | 0.5 |
| Polyglycerin fatty acid ester | 1 |

The saliva secretion amount evaluated as in Example 6 was 12 g. With regards to the bacteria trapping effect and the bacteria coating effect evaluated as in Example 3, the percentages were each 0%.

### Example 8

A lozenge was prepared in the following manner.

### Lozenge (A):

To 46 g of sugar and 29 g of starch syrup, 20 g of water was added, followed by mixing. The resulting mixture was boiled down at 150°C to completely evaporate water from it. The boiled syrup was cooled to about 70°C, to which 12 g of tartaric acid, 12 g of sodium bicarbonate, 0.5 g of menthol and 0.5 g of cineol were added. After mixing, 5 of the resulting mixture was poured into a mold of 20 mm∅ and then cooled.

### Lozenge (B):

### (1) Preparation of the second layer

Sugar (36 g), 22 g of a starch syrup and 7.2 g of water were mixed and the resulting mixture was boiled down at 150°C to completely evaporate water from it. Then the residue was cooled to about 70°C, to which 1 g of menthol and 1 g of cineol were added, followed by mixing. (2) Preparation of the first layer and joining thereof with the second layer

The first layer was prepared by mixing and boiling in a similar manner to that employed for the preparation of Lozenge (A) except that neither menthol nor cineol was added. In a mold of 20 mm∅, 3.5 g of the first layer composition was poured, together with 1.5 of the second layer composition, followed by molding. Upon molding, 1.5 g of the second layer composition was filled in the mold so that it came at the center of the first layer composition. Lozenge (C):

To 46 g of sugar and 30 g of starch syrup, 15 g of water was added. The resulting mixture was boiled down at 150°C to completely evaporate water from it. Then, the residue was cooled to about 70°C, to which 12 g of tartaric acid and 12 g of sodium bicarbonate were added. After mixing, 5 g of the resulting mixture was poured into a mold of 20 mm∅, followed by cooling.

### Comparative Lozenge 1:

Sugar (60 g), 39 g of a starch syrup and 20 g of water were mixed. The resulting mixture was boiled down at 150°C to completely evaporate water from it. Then, the residue was cooled to about 70°C, to which 0.5 g of menthol and 0.5 g of cineol were added. After mixing, 5 g of the resulting mixture was poured into a mold of 20 mm∅, followed by cooling.

Using Lozenges (A) to (C) and Comparative Lozenge 1, the saliva secretion amount, mucin amount and content of a sterilizing component (IgA or lysozyme) in the saliva, and pH in the oral saliva were each measured in the following manner.

### (1) Secretion amount of saliva

Ten adults were asked to wipe off the saliva in the mouth by absorbent cotton prior to the test. Then, they were asked to lick 5 g of each of Lozenges (A) to (C) and Comparative Lozenge 1 and discharge the whole amount of the saliva secreted. The saliva thus secreted was weighed. The secretion amount of the saliva was determined by subtracting, from the whole discharged amount, the weight of the sample in consideration of its foaming amount and then calculating an average of the difference of ten adults.

Secretion amount of saliva = {amount of saliva discharged - (weight of lozenge - stoichiometric production weight of CO₂)}

### (2) Amount of mucin

The amount of mucin in the saliva sampled in (1) was determined in accordance with the Blyscan Assay.

### (3) Amount of IgA or lysozyme in the saliva

By using the saliva sampled in (1), the secretory IgA and lysozyme contents in the saliva sampled in (1) were determined by the Enzyme-linked immunosorbent Assay (ELISA) and the turbidity measuring method using Micrococcus lysodeikticus cells, respectively.

### (4) pH of the oral saliva

The pH of the saliva sampled in (1) was measured by a pH meter using a semiconductor sensor.

The results are shown in FIGS. 3 to 6.

From FIGS. 3 and 4, it has been understood that the amount of the saliva and the amount of mucin in the saliva of the lozenges (Lozenges (A) to (C)) containing an effervescent component or an acid are greater than those of Comparative lozenge 1 free of tartaric acid. This suggests that Lozenges (A) to (C) of the present invention can promote the secretion of the saliva and mucin while keeping the pH of the saliva at healthy pH.

From FIG. 5, it has been understood that Lozenges (A) to (C) of the present invention containing an effervescent component secreted larger amounts of secretory IgA and lysozyme, and secreted saliva having a higher sterilizing power than Comparative lozenge 1.

From the above-described results, it has been found that only Lozenges (A) to (C) of the present invention can increase the amount of the saliva, mucin, IgA and lysozyme without lowering the pH of the saliva in the mouth.

A panel of 20 experts having throat discomfort was asked to slowly lick the lozenges of Examples and Comparative Example one by one after sufficient intervals without crunching them and to evaluate their effects on the throat. The results are shown in Table 2.

**Table 2**

| | Invention lozenge | | Comparative lozenge |
|---|---|---|---|
| | A | B | 1 |
| Free from throat discomfort | 17 | 19 | 4 |
| No change | 3 | 1 | 14 |
| Throat discomfort became severer | 0 | 0 | 2 |

From Table 2, it has been found that many experts felt that Lozenges (A) and (B) of the present invention were effective against the throat discomfort (removed discomfort), compared with Comparative Lozenge 1.

### Example 8

A beverage for drinking after mixing equal amounts of the following components was prepared.

| Component 1 | |
|---|---|
| Water | 42 parts by weight |
| Starch syrup | 50 |
| Citric acid | 7 |
| Sugar | 1 |
| Component 2 | |
| Water | 39 parts by weight |
| Starch syrup | 50 |
| Sodium bicarbonate | 7 |
| Sugar | 1 |
| Menthol | 1 |
| Polyglycerin fatty acid ester | 1 |
| Soybean dietary fiber | 1 |

By effervescence, the secretion of the saliva was promoted and refreshed feeling lasted.

### Industrial Applicability

The throat care agent of the present invention promotes secretion of the saliva, promotes secretion of a large amount of mucin which is a sterilizing component in the saliva, prevents adhesion of bacteria to the throat, removes bacteria from the throat, thereby cleaning the throat and keep the throat refreshed.

## Claims

1. A throat care agent comprising a saliva-secretion promoting component.

2. A throat care agent of Claim 1, wherein the saliva-secretion promoting component is a taste stimulating component, an oral mucosa stimulating component, a salivary gland activating component or a physically stimulating component.

3. A throat care agent of Claim 2, wherein the taste stimulating component is citric acid, isocitric acid, malic acid, acetic acid, oxalic acid, malonic acid, succinic acid, glutaric acid, maleic acid, fumaric acid, aconitic acid, lactic acid, tartaric acid, pyruvic acid, ascorbic acid, aldonic acid, uronic acid, sodium glutamate or inosinic acid.

4. A throat care agent of Claim 2, wherein the oral mucosa stimulating component is allyl isothiocyanate, capsaicin, piperine, sanshool or zingerol.

5. A throat care agent of Claim 2, wherein the salivary glad activating agent is pilocarpine, muscarine, acetylcholine, caffeine, Cola acuminata, Cola nitida, Jaborandi, white birch, honeysuckle, American ginseng, *Houttuynia cordata,* garlic, hibiscus, hop, *Actinidia polygama,* linden or rose hip.

6. A throat care agent of Claim 2, wherein the physiologically stimulating component is an effervescent or a carbon dioxide gas, nitrogen gas or inactive gas filled under high pressure.

7. A throat care agent of any one of Claims 1 to 6, which further comprises a polysaccharide.

8. A throat care agent of Claim 7, wherein the polysaccharide is soybean dietary fiber, guar gum, tara gum, locust bean gun or phosphoric acid polysaccharide.

9. A throat care agent of any one of Claims 1 to 8, which further comprises a component acting on the throat.

10. A throat care agent of Claim 9, wherein the component acting on the throat is a herb, an antitussive and expectorant, chitin, chitosan, honey, propolis, a salivary component, menthol or cineol.

11. A throat care agent of any one of Claims 1 to 10, which is in the form of a lozenge.

12. A throat care agent of any one of Claims 1 to 10, which is in the form of a beverage.

13. A throat care agent of Claim 12, having a viscosity ranging from 2 to 3000 mPa·s.

14. A throat caring method, which comprises administering a saliva-secretion promoting component.

15. Use of a saliva-secretion promoting component for the preparation of a throat care agent.
